Europäisches Patentamt

European Patent Office

Office européen des brevets

(19)

(11) **EP 0 896 672 B1**

(12) **EUROPÄISCHE PATENTSCHRIFT**

(45) Veröffentlichungstag und Bekanntmachung des Hinweises auf die Patenterteilung:
**28.08.2002 Patentblatt 2002/35**

(21) Anmeldenummer: 97922930.9

(22) Anmeldetag: **30.04.1997**

(51) Int Cl.⁷: $G01N\ 33/49$

(86) Internationale Anmeldenummer:
**PCT/EP97/02230**

(87) Internationale Veröffentlichungsnummer:
**WO 97/041431 (06.11.1997 Gazette 1997/47)**

(54) **VERFAHREN UND VORRICHTUNG ZUR MESSUNG DER AGGREGATION DER BLUTPLÄTTCHEN BZW. DER KOAGULATION DES BLUTES**

PROCESS AND DEVICE FOR MEASURING BLOOD PLATELET AGGREGATION OR BLOOD COAGULATION

PROCEDE ET DISPOSITIF DE MESURE DE L'AGREGATION DES PLAQUETTES DU SANG OU DE LA COAGULATION SANGUINE

(84) Benannte Vertragsstaaten:
**DE ES FR GB IT**

(30) Priorität: **30.04.1996 DE 19617407**

(43) Veröffentlichungstag der Anmeldung:
**17.02.1999 Patentblatt 1999/07**

(73) Patentinhaber:
• **Dr. Michael Kratzer GmbH**
**80799 München (DE)**
• **VDG - Von Der Goltz GmbH**
**83370 Seeon (DE)**

(72) Erfinder:
• **KRATZER, Michael**
**D-80799 München (DE)**
• **FREIHERR VON DER GOLTZ, Volker**
**D-83370 Seeon (DE)**

(74) Vertreter: **von Puttkamer, Nikolaus, Dipl.-Ing.**
**Patentanwälte**
**Haft, von Puttkamer,**
**Berngruber, Czybulka**
**Franziskanerstrasse 38**
**81669 München (DE)**

(56) Entgegenhaltungen:
**EP-A- 0 223 244      EP-A- 0 316 599**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 896 672 B1

## Beschreibung

Die Erfindung betrifft ein Verfahren zur Messung der Aggregation der Blutplättchen bzw. der Koagulation des Blutes nach dem Oberbegriff des Patentanspruches 1 sowie eine Vorrichtung zur Durchführung dieses Verfahrens nach dem Oberbegriff des Patentanspruches 12.

Aus der DE 35 41 057 C2 geht ein solches Verfahren hervor, bei dem mit der Hilfe eines in einem Zylinder befindlichen Kolbens Blut in eine Kapillare eingesaugt wird, wobei der Zylinder mit der Kapillare verbunden ist und bei dem der Istdruck gemessen wird, der in dem Raum zwischen dem Kolben und dem eingesaugten Blut herrscht. Dieser Istdruck wird dadurch auf einem Sollwert gehalten, daß der Kolben in Abhängigkeit von der Differenz zwischen dem Istdruck und einem Sollwert bewegt wird. Als Maß für die Aggregation bzw. Koagulation wird die Blutflußmenge in der Kapillare durch Erfassen der Bewegung des Kolbens bestimmt.

Ein Problem eines solchen Verfahrens besteht darin, daß die zu verwendenden Kapillaren äußerst schwierig herzustellen sind, weil der Strömungswiderstand der Kapillare von der vierten Potenz des Radius der Kapillare abhängt.

Dieser liegt in der Größenordnung von 100 µm. Dies bedeutet, daß die Kapillaren mit einem sehr genauen Durchmesser hergestellt werden müssen. Diese Anforderung führt zu hohen Kosten. Ein weiteres Problem besteht darin, daß die Kapillaren verstopfen können, was zu einer Störung der Messungen führt. Zudem muß auch die Innenfläche der Kapillare von höchster Qualität und von Fremdstoffen (z.B. Fett) völlig gereinigt sein, damit die Blutplättchen sich nicht ansetzen, was zu Verstopfungen oder zu unerwünschten Strömungseinflüssen führen würde. Die Ausströmenden müssen gerundet werden, damit die Blutzellen nicht durch Schubspannungen verletzt werden. Neben den hierdurch erhöhten Produktionskosten werden Kosten durch die erforderlichen Qualitätskontrollen verursacht. Die Handhabung von Kapillaren mit derart kleinen Abmessungen für die weitere automatische Verarbeitung ist äußerst schwierig. Die zu den genannten Nachteilen führenden Kapillaren sind aber aus physiologischen Gründen unbedingt notwendig, weil sie den Widerstand der Arteriolen simulieren. Die vorgeschalteten Kapillaren machen den Prozeß der Hämostase effektiver, weil zu Beginn des Meßvorganges bei offener Apertur die Schubspannungen bei großem Flow begrenzt werden und auf diese Weise eine wirksamere Interaktion der Thrombozyten mit dem Kollagen herbeigeführt wird.

Die Aufgabe der vorliegenden Erfindung besteht daher darin, ein Verfahren und eine Einrichtung zur Messung der Aggregation der Blutplättchen bzw. der Koagulation des Blutes zu schaffen bei dem bzw. bei der die auf die Kapillaren zurückzuführenden Probleme vermieden sind.

Diese Aufgabe wird durch ein Verfahren mit den Merkmalen des Patentanspruches 1 und durch eine Einrichtungen mit den Merkmalen des Patentanspruches 13 gelöst.

Der wesentliche Vorteil der Erfindung besteht darin, daß die beim Stand der Technik verwendeten Kapillaren weggelassen werden können, weil diejenigen Bedingungen, durch eine Volumenstrom/Druck-Steuerung ohne Kapillare simuliert werden, die bei der Messung mit einer Kapillare tatsächlich entstehen. Vorteilhafterweise wird die vorliegende Vorrichtung zur Messung der Aggregation der Blutplättchen bzw. der Koagulation des Blutes wesentlichen kostengünstiger, weil die beim Stand der Technik nur sehr aufwendig herstellbaren Kapillaren entbehrlich sind.

Im folgenden werden die Erfindung und deren Ausgestaltungen im Zusammenhang mit den Figuren näher erläutert. Es zeigen:

Fig. 1 ein Schaltbild zur Erläuterung des erfindungsgemäßen Verfahrens;

Fig. 2 die Abhängigkeit zwischen dem Volumenstrom und dem Druck zur Erläuterung des erfindungsgemäßen Verfahrens;

Fig. 3 den zeitabhängigen Druckverlauf an der Apertur;

Fig. 4 eine erfindungsgemäße Einrichtung zur Durchführung des vorliegenden Verfahrens und

Fig. 5 und 6 weitere Einrichtungen zur Durchführung des vorliegenden Verfahrens.

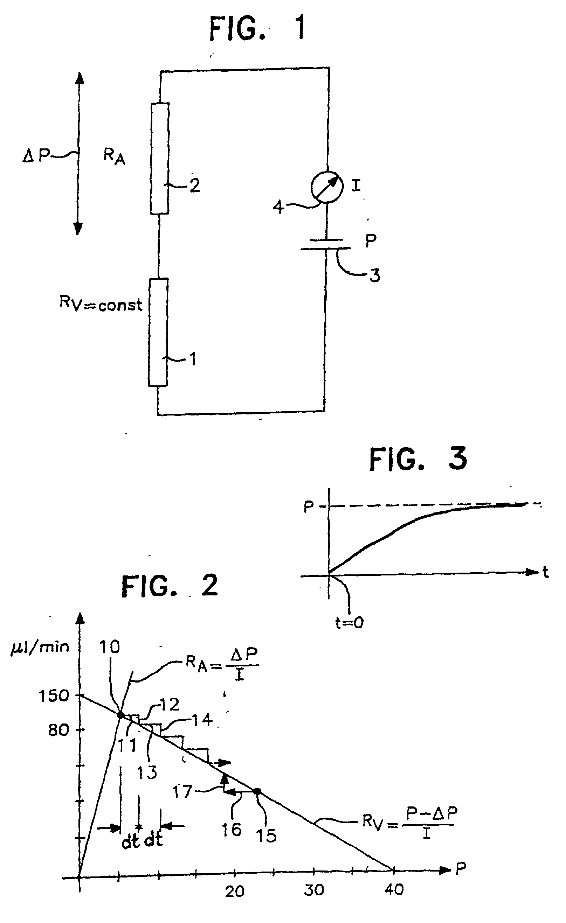

In der Figur 1 ist das Ersatzschaltbild einer bekannten Einrichtung mit einer Kapillare dargestellt, wie sie beispielsweise aus der DE 35 41 057 C2 bekannt ist. Der Strömungswiderstand $R_V$ der Kapillare, der konstant ist, ist mit 1 bezeichnet. $R_A$ bezeichnet den Strömungswiderstand 2 der Apertur, I den Volumenfluß und P den von einer Druckquelle 3 erzeugten Druck. Der Volumenfluß I wird durch eine Meßeinrichtung 4 gemessen und angezeigt. An dem die Apertur darstellenden Strömungswiderstand 2 entsteht ein Druckabfall $\Delta P$, der von der Verstopfung der Apertur bzw. von der Thrombusausbildung im Bereich derselben abhängt und von einem minimalen Wert aus (Beginn der Messung zum Zeitpunkt to) bis zum Wert P der Druckquelle 3 ansteigt (Verstopfung der Apertur), wie dies in der Figur 3 dargestellt ist.

Die Volumenflußmenge I wird durch die folgenden Gleichungen bestimmt:

$$I = \frac{P}{R_A + R_V} \qquad (1)$$

$$I = \frac{\Delta P}{R_A} \qquad (2)$$

$\Delta P$ bezeichnet den Druckabfall an der Apertur. Aus dieser Gleichung ergibt sich der Strömungswiderstand 2 der Apertur gemäß der folgenden Gleichung:

$$R_A = \frac{\Delta P}{I} \qquad (3)$$

Durch Einsetzen von $R_A$ gemäß Gleichung 3 in die Gleichung 1 ergibt sich der Druck P nach der folgenden Beziehung:

$$P = \Delta P + R_V \cdot I \qquad (4)$$

Daraus läßt sich die folgende Gleichung für den Volumenstrom I ableiten:

$$I = \frac{P - \Delta P}{R_V} \qquad (5)$$

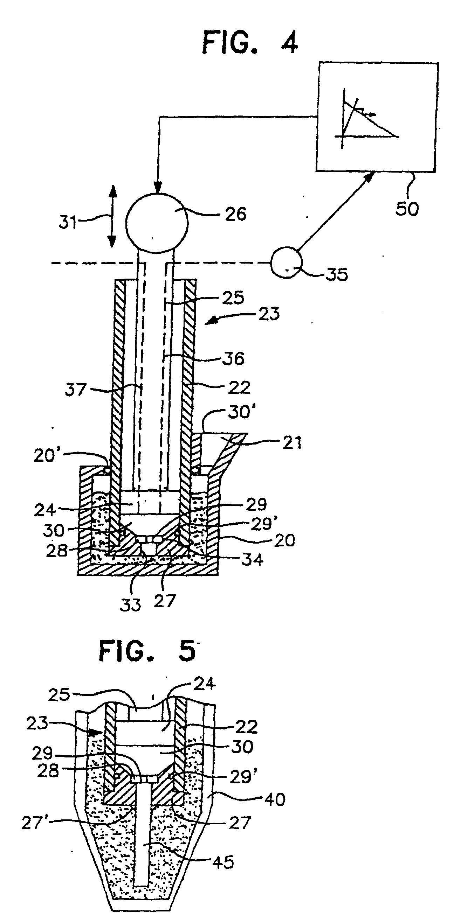

In der Figur 2 sind die Kennlinien $R_V$ und $R_{A(initial)}$ in Abhängigkeit von dem Druck und der Volumenflußmenge dargestellt. Die Funktionen $R_{A(initial)}$ und $R_V$ schneiden sich in einem Punkt 10, der dem Arbeitspunkt kurz nach Beginn der Messung entspricht. Zu Beginn der Messung ist der Widerstand $R_{A(initial)}$ der unverstopften Apertur bekannt. Er ergibt sich experimentell aufgrund der Dimensionen der Apertur 2. Damit ist die Kennlinie $R_A$ festgelegt. Die Kennlinie $R_V$ ergibt sich aus den Dimensionen der zu simulierenden Kapillare, wobei sich bei einem Druck von z.B. 40 mm Hg ein Volumenstrom von z.B. 150 μl/min ergibt. Diese Kennlinien werden in einen Rechner 50 (Figur 4) eingegeben und ergeben den Schnittpunkt 10, der den Bedingungen zu Beginn der Messung entspricht. Wenn sich nun die Apertur 2 fortschreitend verschließt, ist ein entsprechender Druckanstieg $\Delta P$ zu verzeichnen. Der Rechner 50 regelt dann den Volumenstrom I aufgrund der bekannten Kennlinie $R_V$ soweit nach unten, bis I einen der Kennlinie $R_V$ entsprechenden Wert erreicht. Dieses Verfahren wird in vorgegebenen Zeitabständen so oft wiederholt, bis ein vorgegebener Volumenstrom, z.B. der Volumenstrom des Wertes Null, erreicht wird, was einer völligen Verstopfung der Apertur 2 entspricht.

Umgekehrt kann auch so verfahren werden, daß die Regelung den Druck jeweils während einer Zeitperiode konstant hält, und danach, wenn der Volumenstrom um einen vorgegebenen Betrag abgefallen ist, der Druck nachgeregelt wird, bis die Kennlinie $R_V$ erreicht ist.

Beispielsweise zeigt die Figur 3, daß der Druck $\Delta P$ ausgehend von Punkt 10 entlang der Linie 11 ansteigt, woraufhin nach einem Zeitabschnitt dt der Volumenstrom I durch Betätigen des Kolbens 24 in Figur 4 durch den Antrieb 26 unter Steuerung des Rechners 50 entlang der Linie 12 soweit verringert wird, bis die Kennlinie $R_V$ wieder erreicht ist. Nach einem weiteren Druckanstieg $\Delta P$ (Kennlinie 13) wird nach einem Zeitabschnitt dt wieder der Volumenstrom entlang der Linie 14 verringert, bis die Kennlinie $R_V$ erreicht ist. Dies wird solange fortgesetzt, bis letztlich der Wert P bei der Volumenflußmenge Null erreicht wird.

Wenn beispielsweise an einem Punkt 15 der Druck durch teilweise Auflösung der Verstopfung in der Apertur 2 entlang der Linie 16 wieder fällt, wird der Volumenstrom durch das System soweit vergrößert (Linie 17), bis die Kennlinie $R_V$ wieder erreicht ist.

Durch diese beschriebene, fortlaufende Annäherung an die Kennlinie $R_V$ werden bei dem vorliegenden Verfahren die Bedingungen genau simuliert, die bei Vorhandensein einer Kapillare bestehen würden.

Zu Beginn der Messung kann der Rechner 50 die Kennlinie $R_{A(initial)}$ der Apertur 2 selbst ermitteln. Dies erfolgt in einem ersten Schritt dadurch, daß bei einem konstanten Volumenfluß der Druckabfall bei offener Apertur gemessen wird. Dies ist dann auch ein Maß für die Blutviskosität.

Gemäß Figur 4 besteht eine erste Ausführungsform der vorliegenden Einrichtung zur Durchführung des oben beschriebenen Verfahrens aus einem Blutreservoir 20, dem beispielsweise über eine Zufuhröffnung 21 Blut zuführbar ist. Dabei ist die Zufuhröffnung 21 vorzugsweise durch eine Membran 30' verschlossen, die zur Blutzufuhr aus einer Spritze oder dergleichen durch die Spitze derselben durchstoßen wird. Diese Öffnung dient dann zur Belüftung des Raumes 30. In das Blutreservoir 20 wird der Zylinder 22 einer Spritze 23 eingeführt, in dessem Inneren ein Kolben 24 angeordnet ist, der mit der Hilfe eines durch einen Antrieb 26 betätigten Stellgliedes 25 in der Längsrichtung des Zylinders 22 bewegbar ist (Pfeil 31). Eine Abdichtung zwischen dem Zylinder 22 und dem Blutreservoir 20 erfolgt durch eine Dichtung 20'.

In das untere Ende des Zylinders 22, das in das Blutreservoir 20 eintaucht, ist ein Halteteil 27 mit der Hilfe einer Dichtung 29' dicht einsetzbar, wobei das Halteteil 27 eine Durchgangsöffnung 33 aufweist, die an der dem Kolben 24 zugewandten Seite in eine Ausnehmung 34 mündet, in die ein eine Apertur 29 aufweisendes Teil 28 dicht so eingesetzt ist, daß die Durchgangsöffnung 33 der Apertur 29 vorgeschaltet ist. Vorzugsweise sind die Halteeinrichtung 27 und das die Apertur 29 aufweisende Teil 28 als Wegwerfteil in der Form einer kleinen, leicht handhabbaren Einheit ausgebildet. Dies kommt den Lagerproblemen entgegen, die durch die in Klinken vorhandenen relativ kleinen Kühlräumen verursacht werden. Zwischen dem Teil 28. mit der Apertur 29 und dem Kolben 24 befindet sich ein Raum 30, der zur Aufnahme des durch die Apertur 29 hindurchgetretenen Blutes dient. Der im Raum 30 herrschende Druck wird durch einen schematisch dargestellten Sensor 35 gemessen. Die entsprechende Verbindungsleitung zum Raum 30 ist mit 36 bezeichnet.

Es besteht die Möglichkeit, über die Leitung 37 eine bestimmte Flüssigkeit (z.B. NaCl oder andere Substanzen) in den Bereich der Apertur 29 vor der Vornahme einer Messung zu bringen, um das vorzugsweise aus einem Filtermaterial (z.B. Celluloseacetat) bestehende Teil 28 zu tränken. Die Flüssigkeit kann über eine Leitung 37 in den Raum 30 eingebracht werden. Die genannten Leitungen 36, 37 können dicht durch den Kolben 24 oder den Zylinder 22 zum Raum 30 verlaufen.

Vor der Vornahme einer Messung wird dafür Sorge getragen, daß das Blut aus dem Reservoir 20 durch Bewegen des Kolbens 24 in den Durchgang 33 und danach durch die Apertur 29 in den Raum 30 eingezogen wird. Die eigentliche Messung kann dann sofort oder nach einer gewollten Verzögerung beginnen.

Ein wesentlicher Vorteil der Ausführungsform der Figur 4 besteht dabei darin, daß das Ankoppeln des Meßkolbens an das Blut direkt über das kleine Luftpolster des Raumes 30 erfolgt, ohne daß, wie dies beim Stand der Technik der Fall ist, der Apertur 29 störende große Lufträume nachgelagert sind. Durch die genannte Ankoppelung kann erreicht werden, daß der Blutstrom verzögerungsfrei der Bewegung des Kolbens folgt und damit die störenden Benetzungswiderstände zwischen Blut, Apertur und z.B. NaCl sofort überwunden werden können. Ein weiteres Merkmal, um den Luftraum 30 möglichst klein zu halten besteht darin, daß der Sensor 35 und wenigstens ein Teil der Leitung 36 mit vorzugsweise Öl gefüllt werden.

Über die weitere Leitung 37 kann in den Raum 30 auch eine Spülflüssigkeit eingebracht werden, um diesen nach Vornahme einer Messung und vor dem Einsetzen eines neuen Teiles 27 zu spülen. Außerdem kann Luft zum Trocknen des Raumes 30 zugeführt werden.

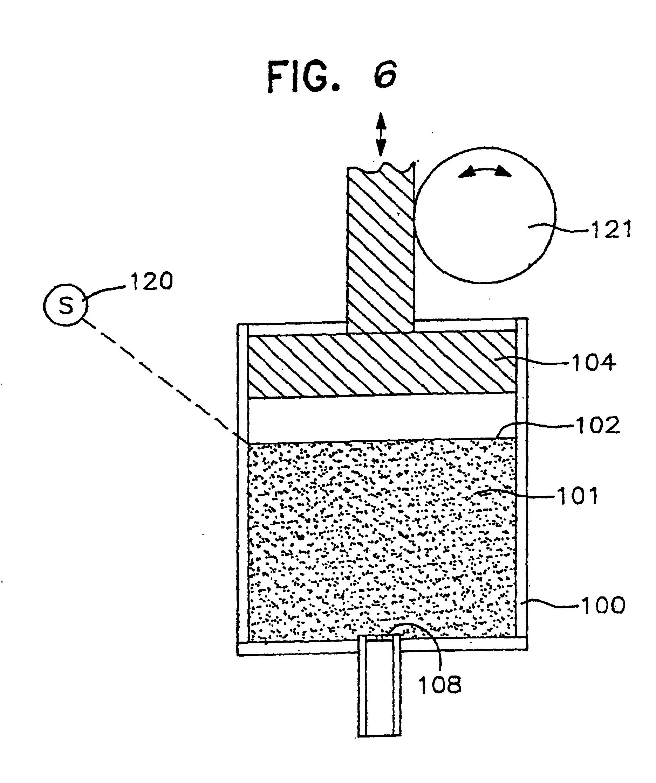

Gemäß Figur 5 ragt ein der Apertur 29 vorgeschaltetes Saugröhrchen 45 in das Blutreservoir 40. Vorzugsweise ist das Röhrchen in den Durchgang 33 gesteckt und mit dem Teil 27 dicht verbunden, zweckmäßigerweise verklebt (Bezugszeichen 27'). Dabei wird darauf hingewiesen, daß das Röhrchen 45 keinen wesentlichen hydrodynamischen Druck erzeugt und nicht mit den Kapillaren des Standes des Technik, die durch die vorliegende Erfindung ja gerade vermieden werden sollen, vergleichbar ist. Das Röhrchen 45, das auch bei der Ausführungsform der Figur 4 zur Anwendung gelangen kann, hat auch eine weitere Aufgabe, die darin besteht, Einflüsse von Scherwirkung darzustellen, die die Blutplättchen beim Entlanggleiten an den Innenumfängen der Arteriolen erleiden. Diese mechanischen Schervorgänge, die für verschiedenartige Untersuchungen von Bedeutung sein können, können tatsächlich nur durch die Anordnung des körperlichen Röhrchens 45 simuliert werden.

Vorzugsweise kann das Blut über das Röhrchen 45 aus einem sogenannten Vacutainer-Röhrchen entnommen werden, das durch einen durchstoßbaren Stopfen verschlossen ist und das Blut unter Unterdruck enthält.

Es wird darauf hingewiesen, daß zur Simulierung unphysiologischer Vorgänge der Widerstand der Kapillare nicht als linear angenommen werden muß. Er kann beispielsweise auch einer quadratischen Funktion folgen, gemäß der er sich gegen Ende der Messung schneller vergrößert als am Angang. Beispielsweise können während des hämostatischen Prozesses starke Hubdrücke zur Simulierung des sogenannten von Willebrand-Faktors von großer Bedeutung sein.

Es wird ferner darauf hingewiesen, daß anstelle der beschriebenen Einsaugung von Blut aus einem Reservoir 20 in die Apertur 29, dieses auch umgekehrt aus dem Zylinderraum der Spritze 23 unter Druck durch die Apertur hindurchgedrückt werden kann.

Vorzugsweise bestehen der Zylinder 22 und das Blutreservoir 20 bzw. 40 aus Kunststoff. Sie werden vorzugsweise zusammen mit dem das Teil 28 mit der Apertur 29 aufweisenden Halteteil 27 als Wegwerfteil ausgebildet. Dabei kann dieses Wegwerfteil gegebenenfalls auch das Röhrchen 45 umfassen. Ferner ist es denkbar, auch den Kolben 24 und gegebenenfalls auch das Stellglied 25 in das Wegwerfteil zu integrieren.

Wenn der Zylinder 22 beispielsweise aus Glas beseht und nicht in das Wegwerfteil integriert ist, wird er nach jedem Meßvorgang automatisch zusammen mit dem Blutreservoir 20 bzw. 40 gespült. In diesem Fall sind nur das Halteteil 27 mit dem Teil 28 als Wegwerfteil ausgebildet.

Gemäß der schematischen Darstellung der Figur 6 werden definierte Verhältnisse beim Drücken von Blut 101 aus einem Reservoir 100 durch eine Apertur 108 dadurch geschaffen, daß die Messung erst dann eingeleitet wird, wenn der das Blut drückende Kolben 104 die Blutoberfläche 102 berührt. Zu diesem Zweck ist ein Sensor 120 vorgesehen, der ein den Pegel der Blutoberfläche 102 anzeigendes Signal erzeugt, das einen Stellantrieb 121 für den Kolben 104 so betätigt, daß dieser den Kolben 104 bewegt, bis dieser die Blutoberfläche 102 berührt.

**Patentansprüche**

1. Verfahren zur Messung der Aggregation der Blutplättchen bzw. der Koagulation des Blutes, bei dem das Blut eine in einem Teil (28) enthaltene Apertur (29) durchströmt, wobei die Verstopfung der Apertur gemessen wird, **dadurch gekennzeichnet, daß** an der Apertur (29) der sich während der Verstopfung ausbildende Druckabfall ($\Delta$P) in vorbestimmten Zeitabständen (dt) gemessen und der Volumenstrom (I) jeweils so verändert wird, daß er einer vorbestimmten Funktion entspricht, die den Strömungswiderstand ($R_V$) einer der Apertur (29) vorgeschalteten Kapillare simuliert, oder daß während der vorbestimmten Zeitabstände (dt) der Druck ($\Delta$P) konstant gehalten wird und danach, wenn der Volumenstrom (I) um einen Betrag abgefallen ist, nachgeregelt wird, bis er der Funktion entspricht.

2. Verfahren nach Anspruch 1, **dadurch gekennzeichnet, daß** in einem ersten Schritt der Widerstand ($R_{A(initial)}$) der Apertur (29) als Funktion der Dimensionen der Apertur (29) ermittelt wird, daß aus den Dimensionen der zu simulierenden Kapillare der Strömungswiderstand ($R_V$) ermittelt wird, und daß die Messung an einem Punkt (10) beginnt, an dem sich die Kennlinien ($R_{A(initial)}$) und $R_V$) schneiden.

3. Verfahren nach Anspruch 1 oder 2, **dadurch gekennzeichnet, daß** das Blut aus einem Reservoir (20) direkt in die Apertur (29) mit einer der Apertur (29) nachgeschalteten Kolben/Zylinderanordnung (23) in einen dem Kolben (24) der Anordnung (23) im Zylinder (22) der Anordnung (23) vorgeschalteten Raum (30) gesaugt wird, daß der im Raum (30) herrschende Druck mit einem Drucksensor (35) gemessen und in einen Rechner (50) eingegeben wird, in dem die Kennlinien ($R_{A(initial)}$ und $R_V$) gespeichert sind und daß der Rechner (50) in Abhängigkeit von dem im Zeitintervall (dt) jeweils ermittelten Druckabfall $\Delta$P mit einem Antrieb den Kolben (24) im Zylinder (22) zur Veränderungen des Volumenstromes (I) bewegt.

4. Verfahren nach einem der Ansprüche 1 bis 3, **dadurch gekennzeichnet, daß** als Antrieb (26) ein über ein Stellglied (25) an den Kolben (24) gekoppelter Schrittmotor verwendet wird.

5. Verfahren nach einem der Ansprüche 1 bis 4, **dadurch gekennzeichnet, daß** als Teil (28) ein die Apertur (29) enthaltendes Filterteil verwendet wird.

6. Verfahren nach Anspruch 5, **dadurch gekennzeichnet, daß** als Filterteil eine Celluloseacetatfilter verwendet wird.

7. Verfahren nach einem der Ansprüche 1 bis 6, **dadurch gekennzeichnet, daß** Filterteil vor Beginn der Messung und dem Einsaugen des Blutes in die Apertur (29) mit einer Flüssigkeit benetzt wird.

8. Verfahren nach Anspruch 7, **dadurch gekennzeichnet, daß** als Flüssigkeit NaCl verwendet wird.

9. Verfahren nach einem der Ansprüche 1 bis 8, **dadurch gekennzeichnet, daß** der Apertur (29) ein keinen wesentlichen hydrodynamischen Druck erzeugendes Röhrchen (35) vorgeschaltet ist, über das das Blut aus dem Reservoir (40) zur Apertur (29) gesaugt wird, wobei das Röhrchen das Entlanggleiten der Partikel des Blutes an den Innenumfängen von Arteriolen simuliert.

10. Verfahren nach einem der Ansprüche 1 bis 9, **dadurch gekennzeichnet, daß** der Strömungswiderstand ($R_V$) der Kapillare durch eine lineare Kennlinie simuliert wird.

11. Verfahren nach einem der Ansprüche 1 bis 10, **dadurch gekennzeichnet, daß** der Strömungswiderstand ($R_V$) der Kapillare durch eine nichtlineare Kennlinie simuliert wird.

12. Verfahren nach Anspruch 1 oder 2, **dadurch gekennzeichnet, daß** ein durch einen Stellantrieb (121) bewegbarer Kolben (104) das Blut durch die Apertur (108) drückt und daß die Messung begonnen wird, wenn der Kolben (104) die durch einen Sensor (120) detektierte Blutoberfläche (102) berührt.

13. Vorrichtung mit einem durch einen Antrieb (26) in einem Zylinder (22) bewegbaren Kolben (24), wobei der Zylinder (22) einer Apertur (29) nachgeschaltet ist und wobei ein Drucksensor (35) zum Messen des Druckes in dem Raum (30) zwischen der Apertur (29) und dem Kolben (24) vorgesehen ist, zur Durchführung des Verfahrens nach einem der Ansprüche 1 bis 12, **dadurch gekennzeichnet, daß** das die Apertur (29) aufweisende Teil (28) in einem Halteteil (27) angeordnet ist, das dicht in das freie Ende des Zylinders (22) einsetzbar ist und einen der Apertur (29) vorgeschalteten Durchgang (33) aufweist, so daß das Blut aus dem Reservoir (20, 40) durch den Durchgang (23) zur Apertur (29) durch Bewegen des Kolbens (24) in dem Zylinder (22) direkt saugbar ist, ohne daß der Apertur (29) ein störendes Volumen nachgeschaltet ist.

14. Vorrichtung nach Anspruch 13, **dadurch gekennzeichnet, daß** der Kolben (24) über ein Stellglied (25) mit einem Schrittmotor (26) verbunden ist.

15. Vorrichtung nach Anspruch 13 oder 14, **dadurch gekennzeichnet, daß** eine Verbindungsleitung (36) von einem Drucksensor (35) in einen Raum (30) durch den Kolben (24) oder den Zylinder (22) dicht geführt ist, wobei der Raum (30) dem Kolben (24) vorgeschaltet ist.

16. Vorrichtung nach einem der Ansprüche 13 bis 15, **dadurch gekennzeichnet, daß** eine weitere Verbindungsleitung (37) zum Einbringen einer Flüssigkeit in einen Raum (30) durch den Kolben (24) oder den Zylinder (22) dicht geführt ist, wobei der Raum (30) dem Kolben (24) vorgeschaltet ist.

**17.** Vorrichtung nach einem der Ansprüche 13 bis 16, <u>**dadurch gekennzeichnet, daß**</u> das Halteteil (27) und das Teil (28) als Wegwerfteil ausgeführt sind, wobei das Halteteil (27) dicht in das freie Ende des Zylinders (22) einsetzbar ist.

**18.** Vorrichtung nach einem der Ansprüche 13 bis 17, <u>**dadurch gekennzeichnet, daß**</u> eine O-Ringdichtung (29') zum Abdichten des Halteteiles (27) in Bezug auf den Zylinder (22) vorgesehen ist.

**19.** Vorrichtung nach Anspruch 18, <u>**dadurch gekennzeichnet, daß**</u> die O-Ringdichtung (29') in einer Umfangsnut des Halteteiles (27) gehalten ist.

**20.** Vorrichtung nach einem der Ansprüche 17 bis 19, <u>**dadurch gekennzeichnet, daß**</u> auch der Zylinder (22) oder der Zylinder (22) und der Kolben (24) in das Wegwerfteil integriert sind.

**21.** Vorrichtung nach einem der Ansprüche 17 bis 20, <u>**dadurch gekennzeichnet, daß**</u> auch das Blutreservoir (20, 40) in das Wegwerfteil integriert ist.

**22.** Vorrichtung nach einem der Ansprüche 13 bis 21, <u>**dadurch gekennzeichnet, daß**</u> der Apertur (29) ein keinen wesentlichen hydrodynamischen Druck erzeugendes Saugröhrchen (45) vorgeschaltet ist.

**23.** Vorrichtung nach Anspruch 22 in Verbindung mit einem der Ansprüche 17 bis 21, <u>**dadurch gekennzeichnet, daß**</u> auch das Saugröhrchen (45) in das Wegwerfteil integriert ist.

**24.** Vorrichtung nach einem der Ansprüche 13 bis 23, <u>**dadurch gekennzeichnet, daß**</u> das Blut in Vacutainer-Röhrchen enthalten ist, deren Verschluß zur Blutentnahme mit einem Röhrchen durchstoßen wird.

**Claims**

**1.** A process for measuring the aggregation of the blood platelets or coagulation of the blood, in which the blood flows through an aperture (29) contained in a part (28), whereby the clogging of the aperture is measured, **characterised in that** at the aperture (29) the pressure drop ($\Delta P$) that forms during the clogging is measured in predetermined time intervals (dt) and the volume flow (I) is changed in each case so that it corresponds to a predetermined function which simulates the flow resistance ($R_V$) of a capillary provided in front of the aperture (29), **or in that** during the predetermined time intervals (dt) the pressure ($\Delta P$) is kept constant and afterwards, when the volume flow (I) has fallen by an amount, is readjusted until it corresponds to the function.

**2.** A process according to Claim 1, **characterised in that** in a first step the resistance ($R_{A(initial)}$) of the aperture (29) is determined as a function of the dimensions of the aperture (29), **in that** the flow resistance ($R_V$) is determined from the dimensions of the capillary to be simulated, **and in that** the measurement begins at a point (10) at which the characteristics ($R_{A(initial)}$) and $R_V$) intersect.

**3.** A process according to Claim 1 or 2, **characterised in that** the blood is sucked out of a reservoir (20) directly into the aperture (29) using a plunger/cylinder arrangement (23) provided after the aperture (29) into a chamber (30) provided in front of the plunger (24) of the arrangement (23) in the cylinder (22) of the arrangement (23), **in that** the pressure prevailing in the chamber (30) is measured by a pressure sensor (35) and is inputted into a computer (50) in which the characteristics ($R_{A(initial)}$ and $R_V$) are stored, **and in that** by a drive the computer (50) moves the plunger (24) in the cylinder (22) for changes in the volume flow (I) as a function of the pressure drop $\Delta P$ determined in the time interval (dt) in each case.

**4.** A process according to one of Claims 1 to 3, **characterised in that** a stepping motor coupled via an actuating element (25) to the plunger (24) is used as the drive (26).

**5.** A process according to one of Claims 1 to 4, **characterised in that** a filter part containing the aperture (29) is used as part (28).

**6.** A process according to Claim 5, **characterised in that** a cellulose acetate filter is used as the filter part.

**7.** A process according to one of Claims 1 to 6, **characterised in that** the filter part is moistened with a liquid prior to the start of the measurement and the suction of the blood into the aperture (29).

**8.** A process according to Claim 7, **characterised in that** NaCl is used as the liquid.

**9.** A process according to one of Claims 1 to 8, **characterised in that** a tube (35) that does not produce any substantially hydrodynamic pressure is connected in front of the aperture (29), via which tube the blood is sucked out of the reservoir (40) to the aperture (29), whereby the tube simulates the blood particles sliding along the inner peripheries of arterioles.

10. A process according to one of Claims 1 to 9, **characterised in that** the flow resistance ($R_V$) of the capillary is simulated by a linear characteristic.

11. A process according to one of Claims 1 to 10, **characterised in that** the flow resistance ($R_V$) of the capillary is simulated by a non-linear characteristic.

12. A process according to Claim 1 or 2, **characterised in that** a plunger (104) that can be moved by an actuating drive (121) forces the blood through the aperture (108) **and in that** the measurement is started when the plunger (104) touches the surface of the blood (102) detected by a sensor (120).

13. A device having a plunger (24) that can be moved in a cylinder (24) by a drive (26), wherein the cylinder (22) is connected after an aperture (29) and wherein a pressure sensor (35) is provided for measuring the pressure in the chamber (30) between the aperture (29) and the plunger (24), for performing the process according to one of Claims 1 to 12, **characterised in that** the part (28) comprising the aperture (29) is disposed in a retaining part (27), which can be inserted tightly into the free end of the cylinder (22) and comprises a passage (33) provided in front of the aperture (29), so that the blood can be directly sucked out of the reservoir (20, 40) through the passage (23) to the aperture (29) by moving the plunger (24) in the cylinder (22) without there being a disturbing volume after the aperture (29).

14. A device according to Claim 13, **characterised in that** the plunger (24) is connected to a stepping motor (26) via an actuating element (25).

15. A device according to Claim 13 or 14, **characterised in that** a connecting line (36) is tightly guided from the pressure sensor (35) into a chamber (30) through the plunger (24) or the cylinder (22).

16. A device according to one of Claims 13 to 15, **characterised in that** a further connecting line (37) for introducing a liquid into the chamber (30) is tightly conveyed through the plunger (24) or the cylinder.

17. A device according to one of Claims 13 to 16, **characterised in that** the retaining part (27) and the part (28) are designed as disposable parts, with it being possible to insert the retaining part (27) tightly into the free end of the cylinder (22).

18. A device according to one of Claims 13 to 17, **characterised in that** an O-ring seal (29') is provided for sealing the retaining part (27) with respect to the cylinder (22).

19. A device according to Claim 18, **characterised in that** the O-ring seal (29') is retained in a peripheral groove of the retaining part (27).

20. A device according to one of Claims 17 to 19, **characterised in that** the cylinder (22) or the cylinder (22) and the plunger (24) are also integrated into the disposable part.

21. A device according to one of Claims 17 to 20, **characterised in that** the blood reservoir (20, 40) is also integrated into the disposable part.

22. A device according to one of Claims 13 to 21, **characterised in that** a suction tube (45) that does not produce any substantial hydrodynamic pressure is connected in front of the aperture (29).

23. A device according to Claim 22 in conjunction with one of Claims 17 to 21, **characterised in that** the suction tube (45) is also integrated into the disposable part.

24. A device according to one of Claims 13 to 23, **characterised in that** the blood is contained in a vacutainer tube, the seal of which is pierced by a tube for the removal of blood.

**Revendications**

1. Procédé pour mesurer l'agglutination des plaquettes sanguines ou la coagulation du sang, dans lequel le sang s'écoule au travers d'un orifice (29) aménagé dans un élément (28) et on mesure l'obstruction de l'orifice, **caractérisé en ce qu'**on mesure à intervalles de temps (dt) prédéterminés, au niveau de l'orifice (29), la perte de charge ($\Delta P$) qui s'établit pendant l'obstruction et on modifie le débit volumique (I) de telle sorte que celui-ci corresponde à une fonction prédéterminée qui simule la résistance hydraulique ($R_v$) d'un capillaire placé en amont de l'orifice (29) ou que, pendant les intervalles de temps (dt) prédéterminés, la pression ($\Delta P$) reste constante, et lorsque le débit volumique (I) a chuté d'une valeur donnée, celui-ci soit modifié jusqu'à ce qu'il corresponde à la fonction.

2. Procédé selon la revendication 1, **caractérisé en ce qu'**au cours d'une première étape, on détermine la résistance ($R_{A(initial)}$) de l'orifice (29) en fonction des dimensions dudit orifice (29), **en ce qu'**à partir des dimensions des capillaires à simuler on détermine la résistance hydraulique ($R_v$) et **en ce qu'**on commence la mesure en un point (10) où les courbes caractéristiques ($R_{A(initial)}$ et $R_v$) se coupent.

3. Procédé selon la revendication 1 ou 2, **caractérisé en ce qu'**on aspire le sang à partir d'un réservoir (20) directement à travers l'orifice (29) à l'aide d'un ensemble piston/cylindre (23) placé derrière l'orifice (29), dans une chambre (30) prévue dans le cylindre (22) du dispositif (23), en amont du piston (24) du dispositif (23), **en ce qu'**on mesure la pression qui règne à l'intérieur de la chambre (30) à l'aide d'un capteur de pression (35) et on l'introduit dans un calculateur (50) dans lequel sont mémorisées les courbes caractéristiques ($R_{A(initial)}$ et $R_v$) et **en ce que** le calculateur (50), en fonction de la perte de charge ($\Delta P$) déterminée pendant l'intervalle de temps (dt) déplace le piston (24) dans le cylindre (22) aux fins de modifier le débit volumique (I).

4. Procédé selon une des revendications 1 à 3, **caractérisé en ce qu'**on utilise comme moyen d'entraînement (26) un moteur pas à pas qui est couplé au piston (24) par l'intermédiaire d'un organe de réglage (25).

5. Procédé selon une des revendications 1 à 4, **caractérisé en ce qu'**on utilise comme élément (28) un élément de filtre qui contient l'orifice (29).

6. Procédé selon la revendication 5, **caractérisé en ce qu'**on utilise comme élément de filtre un filtre d'acétate de cellulose.

7. Procédé selon une des revendications 1 à 6, **caractérisé en ce qu'**on humidifie l'élément de filtre avec un liquide avant le début de la mesure et l'aspiration du sang à travers l'orifice (29).

8. Procédé selon la revendication 7, **caractérisé en ce qu'**on utilise comme liquide du NaCl.

9. Procédé selon une des revendications 1 à 8, **caractérisé en ce qu'**on dispose en amont de l'orifice (29) un petit tube qui ne génère aucune pression hydrodynamique sensible, au travers duquel le sang est aspiré du réservoir (40) jusqu'à l'orifice (29), le petit tube simulant le glissement des particules sanguines le long des parois intérieures d'artérioles.

10. Procédé selon une des revendications 1 à 9, **caractérisé en ce qu'**on simule la résistance hydraulique ($R_v$) des capillaires par une courbe caractéristique linéaire.

11. Procédé selon une des revendications 1 à 10, **caractérisé en ce qu'**on simule la résistance hydraulique ($R_v$) des capillaires par une courbe caractéristique non linéaire.

12. Procédé selon la revendication 1 ou 2, **caractérisé en ce qu'**un piston (104) déplacé par un actionneur (121) refoule le sang à travers l'orifice (108) et **en ce qu'**on commence la mesure lors de l'entrée en contact du piston (104) avec la surface du sang (102), détectée par un capteur (120).

13. Dispositif comportant un piston (24) déplacé dans un cylindre (22) par un moyen d'entraînement (26), le cylindre (22) étant disposé derrière un orifice (29) et un capteur de pression (35) pour mesurer la pression dans la chambre (30), entre l'orifice (29) et le piston (24), étant prévu, aux fins de mettre en oeuvre le procédé selon une des revendications 1 à 12, **caractérisé en ce que** l'élément (28) comportant l'orifice (29) est disposé dans un élément-support (27) qui est monté étanche dans l'extrémité libre du cylindre (22) et présente un passage (33) situé en amont de l'orifice (29), de telle sorte que le sang puisse être aspiré directement du réservoir (20, 40) à travers le passage (23) jusqu'à l'orifice (29) par déplacement du piston (24) à l'intérieur du cylindre (22), sans qu'un volume perturbateur soit placé derrière l'orifice (29).

14. Dispositif selon la revendication 13, **caractérisé en ce que** le piston (24) est relié à un moteur pas-à-pas (26) par un organe de réglage (25).

15. Dispositif selon la revendication 13 ou 14, **caractérisé en ce qu'**une conduite de liaison (36) mène de manière étanche du capteur de pression (35) à la chambre (30), à travers le piston (24) ou le cylindre (22).

16. Dispositif selon une des revendications 13 à 15, **caractérisé en ce qu'**une conduite de liaison (37) supplémentaire pour amener un liquide dans la chambre (30) traverse de manière étanche le piston (24) ou le cylindre (22).

17. Dispositif selon une des revendications 13 à 16, **caractérisé en ce que** l'élément-support (27) et l'élément (28) sont réalisés sous forme d'unité jetable, l'élément-support (27) pouvant être monté étanche dans l'extrémité libre du cylindre (22).

18. Dispositif selon une des revendications 13 à 17, **caractérisé en ce qu'**il est prévu un joint torique (29') pour assurer l'étanchéité entre l'élément-support (27) et le cylindre (22).

19. Dispositif selon la revendication 18, **caractérisé en**

**ce que** le joint torique (29') est monté dans une gorge périphérique de l'élément-support (27).

20. Dispositif selon une des revendications 17 à 19, **caractérisé en ce qu'**également le cylindre (22) ou le cylindre (22) et le piston (24) sont intégrés dans l'élément jetable.

21. Dispositif selon une des revendications 17 à 20, **caractérisé en ce qu'**également le réservoir à sang (20, 40) est intégré dans l'élément jetable.

22. Dispositif selon une des revendications 13 à 21, **caractérisé en ce qu'**un petit tube (45) qui ne génère aucune pression hydrodynamique sensible est disposé en amont de l'orifice (29).

23. Dispositif selon la revendication 22 en combinaison avec l'une des revendications 17 à 21, **caractérisé en ce qu'**également le petit tube (45) est intégré dans l'élément jetable.

24. Dispositif selon une des revendications 13 à 23, **caractérisé en ce que** le sang est contenu dans de petits tubes sous vide, dont on perce le bouchon au moyen d'une canule pour le prélèvement de sang.

# FIG. 1

# FIG. 3

# FIG. 2

## FIG. 4

## FIG. 5

# FIG. 6